(19) Europäisches Patentamt
European Patent Office
Office européen des brevets

(11) Numéro de publication: **0 020 210**
**B1**

(12) # FASCICULE DE BREVET EUROPEEN

(45) Date de publication du fascicule du brevet: 23.03.83

(51) Int. Cl.³: **B 01 J 20/22**, C 01 B 3/00, C 07 C 13/70

(21) Numéro de dépôt: 80400627.8

(22) Date de dépôt: 09.05.80

(54) **Agents d'adsorption de gaz utiles en particulier pour séparer de l'hydrogène d'une phase gazeuse.**

(30) Priorité: 29.05.79 FR 7913697

(43) Date de publication de la demande: 10.12.80 Bulletin 80/25

(45) Mention de la délivrance du brevet: 23.03.83 Bulletin 83/12

(84) Etats contractants désignés: AT BE CH DE FR GB IT LI LU NL SE

(56) Documents cités:
US-A-3 198 844
JOURNAL OF THE AMERICAN CHEMICAL SOCIETY, vol. 81, nr. 22, November 20, 1959 D. J. CRAM et al.: »Macro Rings. XIX Olefinic Paracyclophanes«, pages 5963—5971
TETRAHEDRON, vol. 27, 1971 Pergamon Press (GB) I. TABUSHI et al.: Preparations and Properties of Tris (2,2,2) Paracyclophane Derivatives«, pages 4845—4853
JOURNAL OF THE CHEMICAL SOCIETY, 1951, pages 201—208
JOURNAL OF THE AMERICAN CHEMICAL SOCIETY, Vol. 82, 1960, pages 5218—23.
JOURNAL OF POL. SC. Polymer letters Ed. Vol. 14, 1976, pages 85—90.

(73) Titulaire: ANVAR Agence Nationale de Valorisation de la Recherche, 13, rue Madeleine Michelis, F-92522 Neuilly-sur-Seine (FR)

(72) Inventeur: Armand, Michel, 10, rue Gabriel Fauré, F-74000 Annecy (FR)
Inventeur: Jeanne, Francis, Les Evéquaux Biviers, F-38330 Saint Ismier (FR)

(74) Mandataire: Bouton Neuvy, Liliane et al, L'Air liquide, Société Anonyme pour L'Etude et L'Exploitation des Procédés Georges Claude 75, Quai d'Orsay, F-75321 Paris Cedex 07 (FR)

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 020 210 B1

BUNDESDRUCKEREI BERLIN

# Agents d'adsorption de gaz utiles en particulier pour séparer de l'hydrogène d'une phase gazeuse

L'invention a pour objet l'utilisation d'agents d'adsorption de gaz utiles en particulier pour séparer des gaz d'un milieu donné, en vue plus spécialement de leur concentration et/ou de leur stockage.

Elle concerne plus particulièrement, en raison de l'intérêt de leurs applications dans de nombreux domaines, l'utilisation adsorbants susceptibles d'emmagasiner de manière sélective des molécules d'hydrogène.

On sait que la molécule d'hydrogène est peu réactive ce qui rend difficile sa rétention dans un système donné.

On a proposé d'emmagasiner l'hydrogène sous forme d'hydrure en le faisant réagir avec un métal tel que le magnésium ou un alliage tel que $LaNi_5$ ou du type Fe-Ti.

La synthèse de tels hydrures nécessite toutefois, de manière désavantageuse, la mise en oeuvre de températures élevées, par exemple, de l'ordre de 350° C pour élaborer l'hydrure de magnésium $MgH_2$.

De plus, une telle synthèse implique la dissociation de la molécule d'hydrogène et présente donc l'inconvénient d'une cinétique de réaction relativement longue.

On a par ailleurs étudié des systèmes d'encapsulation permettant de piéger l'hydrogène dans un réseau cristallin sans qu'il se produise une dissociation de la molécule. On connait ainsi la possibilité de piéger l'hydrogène dans une zéolithe ou dans un clathrate particulier (hydrate mixte de chloroforme). Cependant, ces composés présentent une très faible capacité de stockage de l'hydrogène au voisinage de la température ambiante, ce qui restreint leur champ d'application.

La présente invention vise à réaliser l'inclusion non dissociative de l'hydrogène, et les recherches effectuées ont permis d'établir qu'en choisissant un certain type de composés organiques répondant à une structure moléculaire définie, on disposait de système capable de retenir de manière satisfaisante, dès la température ambiante des molécules de gaz, particulièrement de l'hydrogène, et le cas échéant des molécules plus volumineuses telles l'oxygène ou l'azote.

L'invention a pour objet de nouveaux adsorbants de gaz sous forme moléculaire et plus spécialement l'utilisation de ceux capables d'emmagasiner l'hydrogène.

Elle vise également les formes de mises en oeuvre de ces adsorbants notamment en phase dispersée en solution ou à l'état solide.

Selon un autre aspect, elle vise également les applications des adsorbants dans les secteurs techniques où la séparation plus spécialement d'hydrogène en vue de sa concentration et de son stockage est souhaitée.

Les adsorbants utilisés dans l'invention comportent au moins un composé appartenant à la série des paracyclophanes, ou PCP, c'est-à-dire des composés constitués par des cycles aromatiques reliés en position para par des chaînons linéaires.

Certains dérivés de la série des paracyclophanes, notamment le tris[2,2,2]paracyclophane, le tétrakis[1,1,1,1]paracyclophane et le tris[2,2,2]paracyclophane substitué par le brome ou le radical $-COOH$ sur l'un des cycles aromatiques sont connus par les travaux de I. Tabushi et al, »Tetrahedron« vol. 27, 1971, pages 4845 à 4853. Les dérivés de PCP trimères comportant des chainons $-CH=CH-$ sont aussi connus du »Journal of the American Society« Vol. 81. page 5964.

Conformément à l'invention, on a recours à l'utilisation de PCP constitués par des oligomères cycliques formés de motifs-mères qui l'enchaînent de manière à définir une structure rigide à cavité intramoléculaire sensiblement cylindrique, les dimensions de la section droite de la dite cavité correspondant sensiblement à celles du ou des types de molécules de gaz que les mouvements de rotation des atomes qui constituent les dits motifs ne peuvent entrainer de variations substantielles en volume de la dite cavité jusqu'à une température de l'ordre de 250° C.

Les dimensions de la cavité intramoléculaire, sa bonne adaptation géométrique à la molécule que l'on souhaite inclure ainsi que le caractère rigide du macrocycle peut être avantageusement évalués en utilisant des modèles moléculaires de précision du type CPK (Corey-Pauling-Koltun).

En pratique sont qualifiées de »rigide« des molécules macrocycliques dont la cavité intramoléculaire ne subit, par les mouvement de rotation d'origine thermique au niveau des liaisons covalentes qui réalisent la structure cyclique, au plus que des déformations dimensionelles limitées telles que l'accessibilité des molécules à inclure soit conservée.

Avantageusement, la rigidité de la structure est telle que les variations de la section droite de cavité soient au plus égales à environ 10% en excès ou par défaut jusqu'à la température ci-dessus indiquée.

Pour disposer d'une structure rigide particulièrement appropriée à la rétention de l'hydrogène, on a recours à des PCP trimères, c'est-à-dire des PCP dont le squelette fondamental est formé de 3 motifs, ces motifs, pouvant différer les uns des autres, répondant à la formule RX, dans laquelle R est un composé cyclique aromatique à 6 chaînons, substitué en chacune des positions para par X, ce composé cyclique étant choisi parmi le radical phénylène $-C_6H_4-$, ou un radical aromatique azoté pyridinyle ou pyrymidyle, sous réserve que le ou les atomes d'azote n'occupent pas les positions substituées en para par X; et X est un chaînon linéaire à deux éléments, choisi plus spécialement parmi le radical $-CH_2-CH_2$;

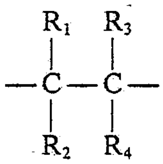 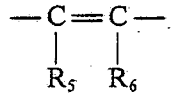 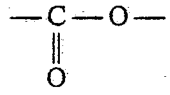 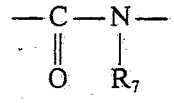 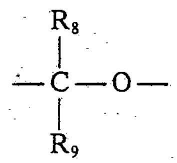

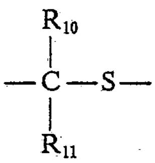 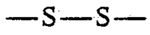 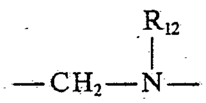 ou 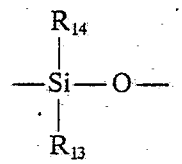

$R_1$ à $R_4$, identiques ou différents les uns des autres, représentant un groupe $C_nH_{2n+1}$ avec $1 \leq n \leq 4$ ou un atome d'halogène choisi parmi F, Cl ou Br, $R_5$ à $R_{14}$ pouvant représenter, en plus des significations prévues pour $R_1$ à $R_4$, un atome d'hydrogène.

Dans les trimères définis ci-dessus, les chaînons de liaison des cycles aromatiques sont des chaînons à deux éléments et en position para. Cette disposition permet d'assurer le maintien des cycles à une distance et selon des angles déterminés les uns par rapport aux autres et ainsi, l'obtention d'une structure stable possédant une rigidité satisfaisante.

L'importance de la nature des chaînons décrits plus haut doit s'apprécier également au niveau de la taille de la cavité qu'ils contribuent à définir. En contrôlant de tels paramètres, il est possible d'obtimiser les interactions ganz-réseau ce qui est particulièrement précieux dans le cas de $H_2$ dont la faible réactivité a été déjà soulignée.

On observera à cet égard que dans les trimères définis ci-dessus, le diamètre intérieur de la cavité est de l'ordre de 1,8 à 2,6 Å, par exemple de 2 Å lorsque X représente le groupe $-CH_2-CH_2$, alors que la molécule de l'hydrogène est un ellipsoïde avec $a=b=2,1$ Å et $c=3$ Å.

Il est clair que l'espace volumique des PCP peut être augmenté en utilisant un chaînon renfermant du soufre ou un groupe siloxane ou alcène. Ceci étant, le choix d'un chaînon donne est fonction également du type de propriétés que l'on souhaite conférer à la molécule de PCP. Aussi, un chaînon substitué en particulier par des groupes alcoyle favorisera notamment l'abaissement du point de fusion des produits. Pour augmenter, notamment, la solubilité des PCP dans les solvants alcooliques ou chlorés, il est intéressant de choisir des chaînons du type ester. Le chaînon siloxane quant à lui permet d'accroître en particulier la stabilité de la molécule de base.

La nature du cycle aromatique R doit être également prise en considération et l'on a ainsi plus particulièrement recours à un cycle aromatique azoté lorsqu'on souhaite disposer d'adsorbants à solubilité accrue notamment dans l'eau.

Des paracyclophanes répondant aux caractéristiques générales définis ci-dessus, mais qui possèdent un logement intramoléculaire de dimension supérieure à celui des trimères considérés et s'avèrent donc plus appropriés à l'introduction de molécules de gaz plus volumineuses que $H_2$ telles que $O_2$ ou $N_2$ renferment au moins un tétramère cyclique formé de motifs mères $R-Y$ dans lesquels R présente la signification déjà donnée et Y est un chaînon à un élément choisi parmi le groupe

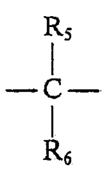

dans lequel $R_5$ et $R_6$ présentent les significations sus-indiquées;

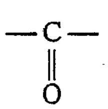 $-O-$ ou $-S-$

Dans des variantes de réalisation, de l'invention, les oligomères cycliques définis ci-dessus comportent un ou plusieurs substituants M sur les cycles aromatiques, ces substituants étant choisis parmi ceux dont l'encombrement ne gêne pas l'accès à la cavité intérieure de la molécule.

Un choix convenable des substituants M permet d'ailleurs d'augmenter le caractère rigide de la cavité en reforçant des barrières de rotation.

Le choix de ces substituants est plus particulièrement guidé par le type de propriété que l'on désire améliorer ou conférer à la molécular de base.

Ainsi, afin d'améliorer notamment le caractère hydrophobe des PCP utilisés dans l'invention, on a recours à des substitions halogène.

Les composés fluorés s'avèrent particulièrement satisfaisants à cet égard, et présentent, en outre,

l'avantege de donner lieu à des PCP possédant une stabilité thermique améliorée.

Le recours à des groupement acides tels que $-COOH$ ou $-SO_3H$ permet d'obtenir une meilleure solubilisation des PCP en milieu aqueux. On augmente aussi la solubilisation en milieu aqueux des PCP en introduisant des groupement azotés et plus spécialement des cations ammonium du type $-N(R_{15})_3^+$, $R_{15}$ représentant un radical $C_{n'}$ $H_{2n'+1}$ avec $0 \leq n' \leq 2$. Les substituants M sont avantageusement présents à raison de 1 ou 2 par molécule de PCP et même jusqu'à 4 par cycle aromatique dans le cas plus particulièrement de F.

Comme substituant M, on met également avantageusement en oeuvre un groupe capable de réagir aisément avex d'autres composés chimiques et/ou de se polymériser.

Un groupe insaturé éthylénique tel que le groupe vinyle présente à cet égard un grand intérêt, compte tenu de son obtention aisée, selon les méthodes classiques.

Conformément à un aspect avantageux de l'invention, permettant de disposer aisément d'adsorbants sous forme macromoléculaire, ce qui élargit considérablement leur champ d'application, on utilise des dérivés de PCP rattachés à une chaîne polymère. Cette chaîne peut résulter de la polymérisation d'un substituant polymérisable du PCP et correspondre par exemple à une chaîne du type poly (vinyle), éventuellement copolymérisée avec des monomères tels que le fluorure de vinylidène ou un acétal de chlorure de vinyle. Il peut s'agir également de dérivés constitués par des PCP greffés à une chaîne polymère qui constitue en quelque sorte une trame sur laquelle sont fixés les molécules de PCP. Une telle chaîne est par exemple constituée par du poly(p-vinyl), p'-méthyl-PCP benzène

$$-CH_2-\overset{|}{C}H-C_6H_4-CH_2-(PCP)$$

La représentation d'une manière générale d'une liaison groupe chimique $-\!\!($PCP) signifie, dans la description et les revendications, que le groupe chimique en question occupe l'une des positions libres de l'un des cycles aromatiques du PCP.

La préparation des PCP ci-dessus s'effectue selon les techniques connues.

D'une manière générale, on met en oeuvre comme composé de départ le dérivé aromatique comportant le noyau désiré, substitué en chacune des positions para par un groupement fonctionnel appropié à l'obtention des chaînons de liaisons souhaités pour le PCP, ce noyau étant le cas échéant substitué sur les autres positions libres du cycle par l'un des éléments M susindiqués ou par un substituant capable de conduire à un tel élément.

Pour la synthèse des PCP trimères dans lesquels les noyaux aromatiques sont réunis par des groupements éthylène, on fait par exemple réagir un halogénure de para-xylylène en présence de tétra phényléthylène et de sodium dans un milieu solvant tel que le tétrahydrofuranne.

Une variante préférée de préparation de dérivés de ce type est basée sur la pyrolyse du para-xylène est décrite par Errede et al dans JACS, vol. 82, 5218 – 23 (1960).

L'obtention de trimères de PCP dont les noyaux sont reliés par les chaînons fonctionnels X décrits ci-dessus relève des pratiques courantes au laboratoire pour la synthèse de tels groupes.

En ce qui concerne la préparation des PCP tétramères, elle est avantageusement réalisée selon une réaction de type Friedel et Crafts en présence d'un solvant inerte chloré.

La synthèse de ces trimères ou tétramères s'accompagne généralement de la formation de composés linéaires et/ou d'oligomères supérieurs. L'élimination de ces produits est avantageusement effectuée par chromatographie sur colonne.

L'adsorption sur gel de silice s'avère généralement satisfaisante et la séparation souhaitée est réalisée à l'aide d'éluants tels que le benzène ou l'hexane, éventuellement en opérant un gradient de concentration.

L'étude du comportement vis-à-vis des gaz des PCP répondant aux caractéristiques définis ci-dessus a montré que leur logement interne constitue une structure hôte vis-à-vis des molécules de gaz de dimensions sensiblement voisines à celles du logement. Les essais relatifs à l'adsorption de $H_2$ par les trimères cycliques seront plus particulièrement considérés dans ce qui suit. Mais il est clair que les tétramères cycliques dont la cavité interne possède une dimension supérieure permettant la rétention de molécules plus volumineuses et telles que celles de $O_2$ ou $N_2$.

D'une manière générale, on a constaté que l'hydrogène vient avantageusement se loger dans la cavité interne des trimères cycliques sans dissociation de la molécule, à des températures voisines de l'ambiante et en opérant dans une gamme de pression facilement accessible, de 5 à 100 bars et notamment de 5 à 50 bars.

Ces PCP s'avèrent donc particulièrement précieux pour séparer, dans des conditions opératoires aisées, de l'hydrogène de milieux le renfermant ou pour isoler des phases le renfermant d'autres milieux et ce, dès lors que la cavité des PCP ne peut admettre de molécules plus volumineuses.

En raison de l'effet de tamisage ainsi obtenu, les adsorbants utilisés dans l'invention trouvent des applications dans de nombreux secteurs de l'industrie où une séparation sélective d'hydrogène est souhaitée. Les quantités d'adsorbants à mettre en oeuvre pour obtenir l'efficité désirée sont aisément déterminables dans chaque cas.

Ces adsorbants sont donc utilisés avec avantage pour augmenter la teneur en hydrogène d'une phase ainsi qu'à des fins de purification. En permettant l'élimination de molécules plus volumineuses telles que $O_2$, CO ou encore $H_2O$ qui peuvent accompagner $H_2$, ces adsorbants jouent le rôle d'agents de transfert de matière en modifiant la composition d'un système donné.

Il est également intéressant de mettre en oeuvre ces adsorbants ent tant qu'agents protecteurs des composés de stockage d'hydrogène tels que les hydrures métalliques. L'effet de tamisage des adsorbants trimères empêchee le contact des hydrures avec l'oxygène et la vapeur d'eau. Ils participent de plus de manière avantageuse au processus de rétention de l'hydrogène.

Leurs applications électrochimiques revêtent également une grande importance.

Les adsorbants décrits sont ainsi utilisables pour l'élaboration d'électrodes à gaz et permettent d'isoler de l'hydrogène, contenu dans un mélange quelconque, d'un milieu électrolytique, par exemple aqueux.

De telles électrodes formées d'un métal poreux et des matériaux utilisés de manière classique pour leur fabrication, mais comportant sur au moins une partie de leur surface und adsorbant décir, par exemple avanteusement sous forme pelliculaire, entrent donc dans le cadre de l'invention.

Les adsorbants décrits sont utilisables de manière anlogue dans des piles à combustibles, fonctionnant, par exemple, avec du méthanol et donnant donc lieu à la formation d'oxyde de carbone et d'hydrogène, ainsi que dans des générateurs électrochimiques dont le fontionnement fait intervenir un équilibre avec des hydrures tels que le système $NiO_2\ H_x - H_2$ avec $1 < x < 2$ ou $A_gO_x - H_2$ avec $0 < x < 1$. La forme de mise en oeuvre des adsorbants dépend naturellement du type d'application envisagé. On peut souhaiter utiliser les adsorbants sous forme solide. Etant donné toutefois le réseau cristallin compact des PCP qui les constituent l'accès à leur cavité est alors rendu difficile.

Il est donc préférable de les mettre en oeuvre en phase dispersée.

Il peut s'agir d'une dispersion ou dissolution dans un liquide et notamment dans un liquide visqueux, plus spécialement, dans un matériau de membrane classique, selon des proportions permettant l'adsorption désirée. Dans une telle forme de mise en oeuvre le PCP est avantageusement greffé à une chaîne polymère par radiation ou par voie chimique par exemple au poly(p-vinyl, p'-chlorométhyl-benzène) ou encore utilisé sous forme polymère par exemple de polyvinyl.

—(PCP)

Il est également intéressant de les incorporer, en quantité efficace selon l'application visée, plus spécialement par copolymérisation dans une phase dispersée amorphe d'un matériau macromoléculaire. De tels copolymères sont utilisables sous forme de billes ou granulés, par exemple dans une colonne de séparation ou encore sous forme de membranes ou films.

D'autres caractéristiques et avantages de l'invention apparaîtront au cours de la description des exemples qui suivent.

Exemple 1

Préparation du tris[2,2,2]paracyclophane ou $2^3$PCP de formule

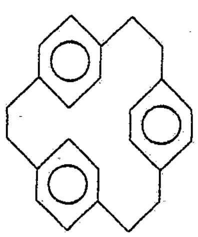

En opérant selon la méthode de Tabushi et al, décrite dans Tetrahedron vol. 27, p 4845-53, on effectue la synthèse du $2^3$PCP en mettant en oeuvre du chlorure de p-xylylène $ClCH_2-C_6H_4-CH_2Cl$, du tétraphényléthylène $(H_5C_6)_2-C=C-(C_6H_5)_2$ et du sodium en poudre.

On ajoute 10 g de sodium en poudre à une solution de 1 g de tétraphényléthylène dans un litre de tétrahydrofuranne (THF). On chauffe le mélange à reflux en agitant énergiquement. Après développement d'une couleur violette du mélange réactionnel, on ajoute 25 g de chlorure de p-xylylène dans 200 ml de THF anhydre. On conduit cette addition à une vitesse permettant le maintien de la couleur violette du mélange réactionnel, ce qui correspond à peu près à une durée de 48 heures. On filtre ensuite le mélange et, en évaporant le filtrat, on obtient un solide qu'on chromatographie sur gel de silice en pratiquant un gradient d'élution hexane-benzène. On récupère le $2^3$PCP recherché avec un rendement voisin de 12%. PF: 168° C (n-hexane).

Caractéristiques physiques:

IR(KBr) $cm^{-1}$: 3020, 2925, 2850, 1515, 1440, 795, 750.
RMN ($CCl_4$, TMS, 33° C) $\delta$ $CH_2$ = 2,93 (s); $\delta_{arom}$ = 6,62 (s)
UV (n hexane); $\lambda$nm(log$\varepsilon$): 276 (2,90); 268 (2,97); 262 (2,85)
SM; m/e: 312 [M+]; 104 ($CH_2-C_6H_4-CH_2$).

Selon un autre mode opératoire, en effectue la synthèse du $2^3$ PCP en pyrolysant du p-xylène conformément à la technique d'Errede et al basée sur la réaction suivante:

$$CH_3-C_6H_4-CH_3 \xrightarrow{\text{pyrolyse}} \cdot CH_2-C_6H_4-CH_2 \cdot \longrightarrow (CH_2-C_6H_4-CH_2)_3$$

On prépare une solution environ 0,10 molaire de p-xylylène dans de l'hexane par pyrolyse rapide de p-xylène et condensation de pyrolysat produit dans 4 l. d'hexane maintenu à −78° C.

Les conditions de la pyrolyse sont les suivantes température: 1065 ± 5° C; pression: 433,28 Pa; temps de séjour: 0,0041 ± 0,003 sec.

On chauffe ensuite la solution de −78° C à la température ambiante.

On élimine par filtration le précipité insoluble qui se forme et on évapore à sec le filtrat. Le traitement du filtrat permet de récupérer 570 g de $2^3$PCP de PF = 167° C.

La synthèse des différents PCP substitués définis plus haut est naturellement possible selon cette méthode. A titre illustratif on rapporte plus loin dans l'exemple 13 des résultats d'essais relatifs à l'adsorption d'hydrogène par le $2^3$PCP en phase dispersée en solution.

## Exemple 2

Préparation du $2^3$vinyl-PCP

$$CH_2{=}CH-(PCP)$$

On effectue cette synthèse selon un processus comportant les étapes:

a: d'acétylation du $2^3$PCP obtenu dans l'exemple 1 à l'aide d'une réaction de type Friedel et Crafts;
b: de réduction du dérivé acétylé obtenu;
c: de déshydratation de l'alcool formé ce qui conduit au dérivé vinyle recherché.

### a: acétylation.

On opère comme indiqué par Tabushi, p. 4852, de la référence précitée.

On constitue un mélange de 500 mg de $2^3$PCP et de 438 mg de chlorure d'aluminium $AlCl_3$ dans 4 ml de disulfure de carbone $CS_2$. On soumet ce mélange à agitation et on ajoute, goutte à goutte, en une heure, une solution de 158 mg d'anhydride acétique $(CH_3CO)_2O$ dans 1 ml de $CS_2$.

Après une agitation supplémentaire de deux heures environ, on verse la solution réactionnelle sur un mélange de glace et d'acide chlorhydrique HCl. On extrait avec du chloroforme $CHCl_3$ et on lave l'extrait chloroformique avec de l'eau, puis une solution saturée de carbonate acide de sodium $NaHCO_3$ et enfin une solution saturée de chlorure de sodium NaCl.

Après séchage et concentration de la solution, on soumet le mélange réactionnel à une chromatographie sur colonne de gel de silice. On élue tout d'abord avec de l'éther de pétrole puis du benzène. Par évaporation des fractions éluées avec le benzène, on récupère le dérivé de PCP recherché substitué par un groupe acétyle, de PF 80−81° C.

### b: réduction

On transforme le groupe acétyle en un groupement fonctionnel d'alcool primaire en opérant selon les techniques classiques, et notamment en faisant réagir le dérivé acétyle avec un hydrure double tel que le borohydrure de sodium, ou l'hydrure mixte d'Al ou de Li. On recueille l'alcool correspondant formé.

### c: déshydratation

L'alcool obtenu est soumis à une réaction de déshydratation en opérant par exemple en milieu acide selon les modes opératoires habituels de synthèse d'alcènes, notamment avec $KHSO_4$.

Exemple 3

Préparation du poly-vinyl-2$^3$PCP comportant le motif

$$-(CH_2-\underset{\substack{|\\(PCP)}}{CH})_n-$$

Le monomère vinylique tel qu'obtenu selon l'exemple 2 est soumis à une réaction de polymérisation radicalaire. Selon les techniques habituelles dans ce domaine, telles que celles utilisées pour préparer du polystyrène, on produit les radicaux libres à l'aide d'initiateurs tels que des peroxydes ou des composés azoïques soumis à une décomposition thermique.

Exemple 4

Préparation de dérivés de PCP dans lesquels les cycles atomatiques sont reliés par des groupes éthylène $-CH=CH-$. En opérant conformément à l'une des méthodes de synthèse générale des alcènes, on met en oeuvre, comme produit de départ un dérivé de PCP comportant des chaînons de liaison monohalogénés, par exemple, monobromés et répondant donc à la formule $-CH_2-CHBr-$, et on le soumet à une réaction de déshydrohalogénation en milieu alcoolique en présence d'une base forte telle que la potasse.

Exemple 5

Préparation de dérivés de PCP comportant des chaînons de liaison du type ester répondant donc à la formule $-CO-O-$.

On chauffe de l'acide acétylsalicylique à 200° C environ ce qui conduit à l'acide paraacétoxybenzoïque.

Par chauffage à sec, cet acide se trimérise en formant l'ester recherché avec élimination d'acide acétique. On sépare cet ester du mélange réactionnel par chromatographie en phase liquide sous haute pression. On opère également avanteusement selon la technique de Wilson Baker dans J.-Chem. Soc. 1951, p. 201.

Exemple 6

Préparation de dérivés de PCP dans lesquels les cycles aromatiques sont reliés par des groupes amides de formule $-CO-NH-$ ou $-CO-N-R_7$.

On opère comme dans l'exemple 5 mais en partant de l'acide p-acétamido-benzoïque, le groupement acétamide étant, le cas échéant, substitué par $R_7$.

Exemple 7

Préparation de dérivés de PCP dans lesquels les cycles aromatiques sont reliés par des groupes éther $-CH_2-O-$.

On opère comme dans l'exemple 5 mais en partant de p-chlorométhylphénol et en opérant en milieu basique. La condensation du dérivé phénolique s'accompagne d'une élimination d'acide chlorhydrique.

Exemple 8

Préparation du tétrakis[1,1,1,1]paracyclophane ou 1$^4$-PCP de formule

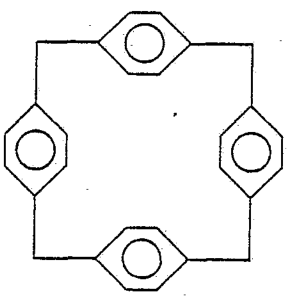

On soumet un p-Halogénométhyl-benzène, notamment le chlorure de benzyle à une réaction du type Friedel et Crafts, en opérant en milieu fortement dilué dans un solvant inerte chloré, tel que le dichlorobenzène, ou le chlorure de méthylène, en présence de trichlorure d'aluminium $AlCl_3$ et/ou de trifluorure de bore $BF_3$. Par élimination d'acide chlorhydrique, on obtient la condensation désirée en tétramère. Dans une variante, on fait réagir du chlorure de p-Xylylène avec du benzène.

Exemple 9

Préparation de dérivés de PCP dans lesquels les cycles aromatiques sont reliés par des groupes silanol

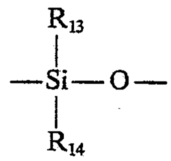

Pour obtenir ce type de composés, on condense en milieu acide ou basique un dérivé de phénolsilanol du type

$HO-C_6H_4-Si(R_{13}, R_{14})-OH$.

Exemple 10

Préparation de dérivés de PCP dans lesquels les cycles aromatiques sont reliés par un pont disulfure.

On procède selon les techniques classiques en oxydant le p,p'-Thiol $HS-C_6H_4-SH$ à l'aide d'un agent oxydant doux tel que les halogènes, $H_2SO_4$ ou analogues.

Exemple 11

Préparation de dérivés de PCP comportant des chaînons

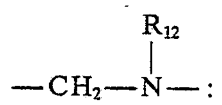

On effectue la condensation de molécules de p-amino, p'-chlorométhyl du type $R_{12}NH-C_6H_4-CH_2Cl$ dans un solvant inerte.

Exemple 12

Préparation de dérivés de PCP comportant des chaînons $-CH=CH-$. Selon une variante du procédé décrit dans l'exemple 4, on prépare lesdits PCP en opérant conformément à la méthode décrite par D. J. CRAM et K. C. DEWHIRST de J. A. C. S. 1969 – 81 p. 5963. On mélange 3,1 g de $2^3$PCP et 10,8 g de N-bromosuccinimide dans 300 ml de $CCl_4$. On ajoute une petite quantité de peroxyde de benzoyle, on agite et on chauffe à reflux pendant environ 14H. Le mélange est filtré, concentré, puis dilué avec du pentane. On récupère 7,2 g de produit brut de PF 250 – 280° C (rendement 91%). Pour l'analyse, on chromatographie le produit et on le recristallise dans un mélange de benzène et d'hexane, ce qui donne un isomère de PF (décomp), 303° C. On mélange 4,5 g de dérivé hexabromé, 20 g de zinc activé avec $NH_4Cl$ et 600 ml d'éthanol. On porte le mélange à reflux sous azote pendant environ 14h. puis on filtre pour éliminer le zinc.

On chromatographie le solide récupéré sur une colonne d'alumine neutre en utilisant comme éluant du pentane. On récupère 820 mg de produit de PF 128 – 131° C (rendement 47%). Une purification par cristallisations répétées dans un mélange éthanol-eau donne un produit de PF 136 – 136,8° C.

Exemple 13

Le mode opératoire général utilisé est le suivant: une solution de $2^3$PCP dans un solvant organique est saturée par de l'hydrogène dans des conditions de température et de pression déterminées. La solution est ensuite rapidement gelée à 77K et l'hydrogène gazeux de l'enceinte est évacué sous vide

primaire (13,33 à 0,13 Pa).

Après réchauffage à température ordinaire, on extrait l'$H_2$ dissous par évaporation du solvant puis condensation en piège froid. On mesure le volume de gaz extrait à l'aide d'une pompe de Töpler fonctionnant avec du mercure et d'une burette à gaz. Dans les essais dont les résultats sont rapportés ci-après, on a utilisé comme solvant un solvant commun à la fois à l'hydrogène et au dérivé des PCP tel que le dichlorométhane $CH_2Cl_2$.

Dans le tableau qui suit, on compare les mesures de gaz extrait en présence d'une quantité connue de $2^3$PCP à celles obtenues d'une part à l'aide du solvant seul, d'autre part, d'une solution de même molarité de l'homologue non cyclique du $2^3$PCP à savoir le 1,4 bis (p-tolyléthyl)-benzène ou BTB. Ces mesures exprimées en $cm^3$, ramenées à des conditions de température et de pression normales, sont données en fonction de la nature de la solution utilisées et des conditions de saturation mises en oeuvre. On indique à ce propos dans chaque cas le volume total d'hydrogène adsorbé par la solution et le volume calculé correspondant à l'hydrogène piégé par le macrocycle de PCP, ce volume étant obtenu par différence avec l'expérience témoin. Le taux de complexation ou de remplissage des cavités est également rapporté et correspond au rapport du nombre de molécules de gaz piégé ou complexé au nombre de molécules de macrocycles présents en solution.

| Nature de la solution | | | Condition de saturation | | | V ($cm^3$) | | Taux de complexa-tion |
|---|---|---|---|---|---|---|---|---|
| V (ml) $CH_2Cl_2$ | Masse $2^3$PCP | (mg) BTB | P (bars) | T (°C) | t (mn) | $H_2$ mesuré | piégé | $\Theta \times 100$ |
| 1 | 0 | 0 | 50,45 | 24 | 120 | 2,31 | – | – |
| 1 | 0 | 88,47 | 50,43 | 25 | 60 | 2,48 | – | – |
| 1 | 0 | 88,47 | 50,49 | 22 | 60 | 2,53 | – | – |
| 1 | 58,5 | 0 | 10,50 | 21,5 | 60 | 0,93 | 0,41 | 9,8 |
| 1 | 89,28 | 0 | 20,53 | 20 | 60 | 1,65 | 0,64 | 10 |
| 1 | 58,5 | 0 | 30,47 | 23 | 60 | 2,41 | 0,90 | 21,4 |
| 1 | 58,5 | 0 | 50,47 | 23 | 60 | 3,58 | 1,08 | 25,6 |

L'examen des résultats de ce tableau met en évidence l'inclusion de $H_2$ dans les logements du $2^3$PCP en solution et ce de manière avantageuse à des températures de l'ordre de l'ambiante et sous des pressions facilement accessibles de 10 à 15 bars environ. La comparaison des résultats obtenus avec le $2^3$PCP et son homologue linéaire est également intéressante et met bien en évidence que l'adsorption d'hydrogène s'effectue par pénétration du gaz dans le logement intramoléculaire du PCP.

Le taux de complexation $\Theta$ correspond à un rapport molaire entre le nombre de molécules d'hydrogène complexées sur le nombre de molécules de complexant, c'est-à-dire de cyclophane en solution. Cette fraction est multipliée par 100 pour exprimer un pourcentage.

**Revendications**

1. Adsorbants des gaz comportant au moins un paracyclophane (ou PCP) trimère formé de trois motifs, ces motifs pouvant différer les uns des autres, répondant à la formule RX, dans laquelle R est un composé cyclique aromatique à 6 chainons, substitué en chacune des positions para par X, ce composé cyclique étant choisi parmi le radical phénylène $-C_6H_4-$, ou un radical aromatique azoté pyridinyle ou pyrimidinyle, sous réserve que le ou les atomes d'azote n'occupent pas les positions substituées en para par X; et X est un chaînon linéaire à deux éléments, choisi plus spécialement parmi le radical

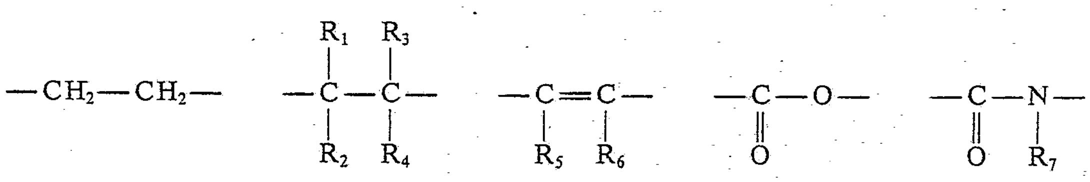

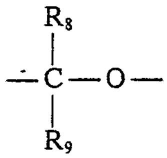 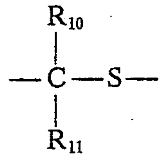 —S—S— 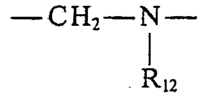 ou 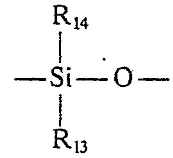

$R_1$ à $R_4$ identiques ou différents les uns des autres représentant un groupe $C_nH_{2n+1}$ avec $1 \leq n \leq 4$ ou un atome d'halogène choisi parmi F, Cl, Br, $R_5$ à $R_{14}$ pouvant représenter en plus des significations prévues pour $R_1$ à $R_4$ un atome d'hydrogène, caractérisés en ce que la molécule de PCP est rattachée à une chaîne polymère.

2. Adsorbants selon la revendication 1, caractérisés en ce qu'ils comportent au moins une chaîne polymère du type poly(p-vinyl-PCP).

3. Adsorbants selon la revendication 1; caractérisés en ce qu'ils comportent une trame macromoléculaire sur laquelle sont fixés des motifs PCP.

4. Utilisation en tant qu'adsorbants des gaz des dérivés de paracyclophane ou PCP, constitués par des oligomères cycliques formés de motifs-mères qui s'enchainent de manière à définir une structure rigide à cavité intramoléculaire sensiblement cylindrique, les dimensions de la section droite de la dite cavité correspondant sensiblement à celles du ou des types de molécules de gaz que l'on désire insérer, la rigidité de la structure étant telle que les variations de la section droite de cavité soient au plus égales à environ 10% en excès ou par défaut jusqu'à une température de l'ordre de 250° C.

5. Utilisation selon la revendication 4, caractérisé en que les derivés du paracyclophane comportent au moins un PCP trimère formé de trois motifs, ces motifs pouvant différer les uns des autres, répondant à la formule RX, dans laquelle R est un composé cyclique aromatique à 6 chaînons, substitué en chancune des positions para par X, ce composé cyclique étant choisi parmi le radical phénylène $-C_6H_4-$, ou un radical aromatique azoté pyridinyle ou pyrimidyle, sous réserve que le ou les atomes d'azote n'occupent pas les positions substituées en para par X; et X est un chaînon linéaire à deux éléments, choisi plus spécialement parmi le radical

$-CH_2-CH_2-$ 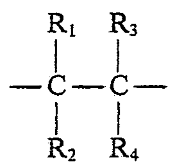 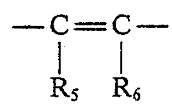 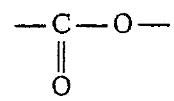 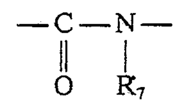

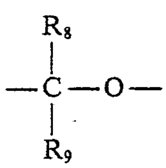 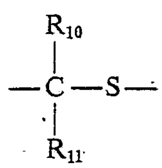 —S—S— 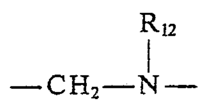 ou 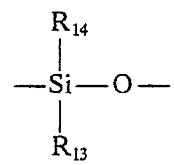

$R_1$ à $R_4$ identiques ou différents les uns des autres, représentant un groupe $C_nH_{2n+1}$ avec $1 \leq n \leq 4$ ou un atome d'halogène choisi parmi F, Cl ou Br, $R_5$ à $R_{14}$ pouvant représenter, en plus des significations prévues pour $R_1$ à $R_4$, un atome d'hydrogène.

6. Utilisation selon la revendication 5, caractérisé en ce que les derivés du paracyclophane comportent au moins un PCP trimère dans lequel X est un chaînon $-CH_2-CH_2-$.

7. Utilisation selon la revendication 5, caractérisé en ce que les derivés du paracyclophane comportent au moins un PCP trimère dans lequel X est un groupement fonctionnel ester.

8. Utilisation selon la revendication 4, caractérisé en ce que les dérivés du paracyclophane renferment au moins un tétramère cyclique formé de motifs-mères R−Y dans lesquels R présente la signification déjà donnée et Y est un chaînon à un élément choisi parmi le groupe

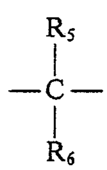

dans lequel $R_5$ et $R_6$ présentent les significations susindiquées;

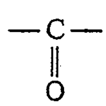 —O— ou —S—

9. Utilisation selon la revendication 5, caractérisé en ce que les dérivés du paracyclophane comportent au moins un PCP trimère formé de trois motifs dont l'un des cycles aromatiques est substitué par au moins un élément M choisi parmi les halogènes, en particulier F, Cl et Br, un groupement acide tel $-COOH$ ou $SO_3H$, un cation ammonium du type $-N(R_{15})_3^+$, $R_{15}$ représentant un radical $C_nH_{2n+1}$ avec $0 \leq n' < 2$, ou un groupe éthyléniquement insaturé, avantageusement le groupe vinyle.

10. Application des adsorbants selon la revendication 5, à la rétention d'hydrogène.

11. Application des adsorbants selon la revendication 8, à la rétention de molécules plus volumineuses que l'hydrogène telles $O_2$ ou $N_2$.

12. Application des adsorbants selon la revendication 10, caractérisé en ce qu'on opère à des températures voisines de l'ambiante et dans un domaine de pression de 5 à 100 bars, et en particulier de 5 à 50 bars.

13. Application des adsorbants selon la revendication 3, caractérisée en ce qu'en vue plus spécialement de la purification d'hydrogène et de sa concentration, on met en contact une phase renfermant une quantité efficace d'au moins l'un des dits adsorbants, avec une phase gazeuse on liquide renfermant de l'hydrogène en mélange avec, par exemple $O_2$, $N_2$ ou $H_2O$.

14. Application selon la revendication 10, caractérisée en ce qu'on met en oeuvre le dit adsorbant, à l'état solide, notamment sous forme de billes, de granulés ou de membranes, dispersé dans une phase amorphe d'un matériau macromoléculaire.

15. Application selon la revendication 10, caractérisé en ce qu'on met en oeuvre le dit adsorbant, à l'état solide, notamment sous forme de billes, de granulés ou de membranes, en dispersion ou dissolution dans un liquide, notamment dans un liquide visqueux, plus spécialement dans un matériau de membrane classique.

16. Application selon la revendication 10, caractérisée en ce que les adsorbants sont utilisés pour l'élaboration d'électrodes à gaz permettant d'isoler de l'hydrogène contenu dans un mélange d'un milieu électrolytique.

17. Application selon la revendication 11, caractérisée en ce que l'adsorbant sous forme pelliculaire constitue au moins une partie de la surface des électrodes.

**Patentansprüche**

1. Gasadsorbentien mit wenigstens einem Paracyclophan(bzw. PCP)-Trimeren aus drei Struktureinheiten, die untereinander verschieden sein können, der Formel RX, worin R eine cyclische aromatische Verbindung mit sechs Kettengliedern bedeutet, die in jeder para-Stellung durch X substituiert sind und unter dem Rest Phenylen-$C_6H_4$- oder dem aromatischen stickstoffhaltigen Pyridinyl- oder Pyrimidinylrest ausgewählt ist, wobei das oder die Stickstoffatome nicht die in para-Stellung durch X substituierten Stellungen einnehmen und X ein lineares zweigliedriges Kettenglied ist, das spezieller unter den Resten

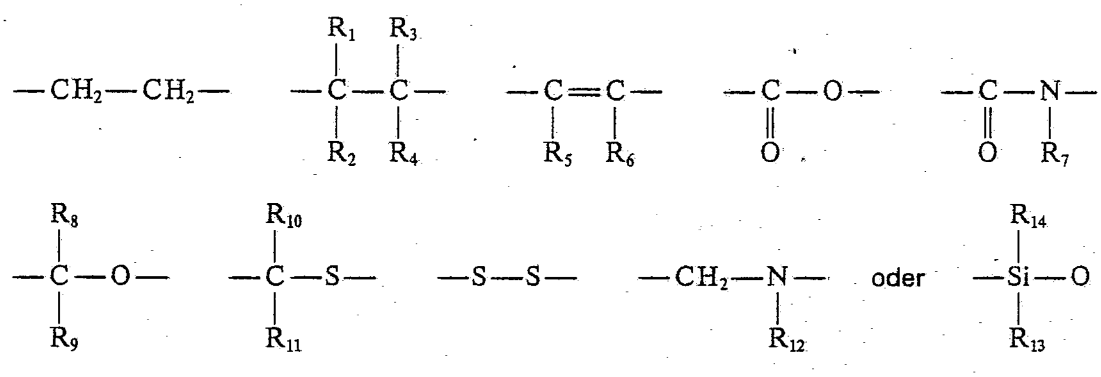

oder

ausgewählt ist, $R_1$ bis $R_4$ gleiche oder untereinander verschiedene Gruppen $C_nH_{2n+1}$, worin $1 = \leq n \leq 4$ bedeuten, oder ein Halogenatom aus der Gruppe F, Cl und Br bedeuten und $R_5$ bis $R_{14}$ außer den oben für $R_1$ bis $R_4$ angegebenen Bedeutungen auch ein Wasserstoffatom bedeuten können, dadurch gekennzeichnet, daß das PCP-Molekül an eine Polymerkette gebunden ist.

2. Adsorbentien nach Anspruch 1, dadurch gekennzeichnet, daß sie wenigstens eine Polymerkette vom Typ Poly-(p-vinyl-PCP) aufweisen.

3. Adsorbentien nach Anspruch 1, dadurch gekennzeichnet, daß sie ein makromolekulares Gerüst besitzen, an welches PCP-Struktureinheiten gebunden sind.

4. Verwendung von Paracyclophan- bzw. PCP-Derivaten, die aus cyclischen Oligomeren bestehen, welche von Grundstruktureinheiten gebildet werden, welche derart verknüpft sind, daß sie eine starre

Struktur eines intramolekularen im wesentlichen zylindrischen Hohlraumes definieren, wobei die Abmessungen des Querschnittes dieses Hohlraumes im wesentlichen denen des oder der Gasmolekültypen entsprechen, die man einzuführen wünscht, und wobei die Starrheit der Struktur derart ist, daß Abweichungen des Querschnittes des Hohlraumes höchstens etwa 10% mehr oder weniger betragen, bis zu einer Temperatur in der Größenordnung von 250° C als Gasadsorbentien.

5. Verwendung nach Anspruch 4, dadurch gekennzeichnet, daß die Paracyclophanderivate wenigstens ein PCP-Trimeres umfassen, das von drei Struktureinheiten, welche voneinander abweichen können, entsprechend der Formel RX gebildet ist, worin R eine cyclische aromatische Verbindung mit sechs Kettengliedern ist, die in jeder para-Stellung durch X substituiert sind, wobei die cyclische Verbindung unter dem Rest Phenylen-$C_6H_4$- oder einem aromatischen stickstoffhaltigen Pyridinyl- oder Pyrimidylrest ausgewählt sind und wobei der oder die Stickstoffatome nicht die durch X in para-Stellung substituierten Stellungen einnehmen und X ein lineares zweigliedriges Kettenglied aus der Gruppe der Reste

$-CH_2-CH_2-$ 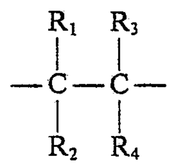 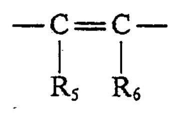 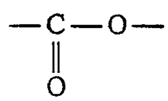 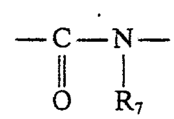

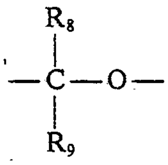 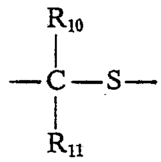 $-S-S-$ 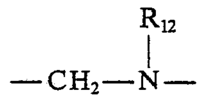 oder 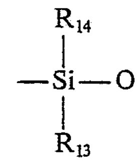

ist,

$R_1$ bis $R_4$ gleiche oder untereinander verschiedene Gruppen $C_nH_{2n+1}$, wobei $1 \leq n \leq 4$ ist, oder ein Halogenatom aus der Gruppe F, Cl oder Br bedeuten und $R_5$ bis $R_{14}$ zusätzlich zu den oben für $R_1$ bis $R_4$ genannten Bedeutungen auch ein Wasserstoffatom bedeuten können.

6. Verwendung nach Anspruch 5, dadurch gekennzeichnet, daß die Paracyclophanderivate wenigstens ein PCP-Trimeres umfassen, worin X ein Kettenglied $-CH_2-CH_2-$ bedeutet.

7. Verwendung nach Anspruch 5, dadurch gekennzeichnet, daß die Paracyclophanderivate wenigstens ein PCP-Trimeres umfassen, worin X eine funktionelle Estergruppe bedeutet.

8. Verwendung nach Anspruch 4, dadurch gekennzeichnet, daß die Paracyclophanderivate wenigstens ein cyclisches Tetrameres einschließen, das aus Grundstruktureinheiten R–Y gebildet ist, worin R die oben angegebene Bedeutung hat und Y ein eingliedriges Kettenglied aus der Gruppe

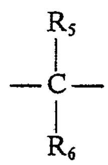

worin $R_5$ und $R_6$ die obigen Bedeutungen haben,

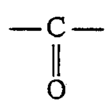 $-O-$ oder $-S-$

bedeutet.

9. Verwendung nach Anspruch 5, dadurch gekennzeichnet, daß die Paracyclophanderivate wenigstens ein PCP-Trimeres umfassen, das von drei Struktureinheiten gebildet ist, von denen einer der aromatischen Ringe durch wenigstens ein Element M aus der Gruppe der Halogenatome, besonders F, Cl und Br, sauren Gruppen, wie $-COOH$ oder $-SO_3H$, Ammoniumkationen vom Typ $-N(R_{15})_3^+$, worin $R_{15}$ einen Rest $C_nH_{2n+1}$ bedeutet, worin $0 \leq n' < 2$ ist, oder äthylenisch ungesättigter Gruppen vorteilhafterweise der Vinylgruppe, substituiert ist.

10. Verwendung von Adsorbentien nach Anspruch 5 zur Zurückhaltung von Wasserstoff.

11. Verwendung von Adsorbentien nach Anspruch 8 zur Zurückhaltung von Molekülen, die voluminöser als Wasserstoff sind, wie $O_2$ oder $N_2$.

12. Verwendung von Adsorbentien nach Anspruch 10, dadurch gekennzeichnet, daß man bei Temperaturen nahe der Umgebungstemperatur und in einem Druckbereich von 5 bis 100 bar, besonders von 5 bis 50 bar arbeitet.

13. Verwendung von Adsorbentien nach Anspruch 3, dadurch gekennzeichnet, daß man spezieller zum Zwecke der Reinigung von Wasserstoff und seiner Konzentrierung eine Phase, die eine wirksame

Menge wenigstens eines dieser Adsorbentien enthält, mit einer gasförmigen oder flüssigen Phase, die Wasserstoff im Gemisch beispielsweise mit $O_2$, $N_2$ oder $H_2O$ enthält, in Berührung bringt.

14. Verwendung nach Anspruch 10, dadurch gekennzeichnet, daß man das Adsorbens in festem Zustand, besonders in der Form von Kugeln, Granalien oder Membranen, in einer amorphen Phase eines makromolekularen Materials dispergiert, verwendet.

15. Verwendung nach Anspruch 10, dadurch gekennzeichnet, daß man das Adsorbens in festem Zustand, besonders in der Form von Kugeln, Granalien oder Membranen, in Dispersion oder Lösung in einer Flüssigkeit, besonders in einer viskosen Flüssigkeit, ganz besonders in einem herkömmlichen Membranmaterial, verwendet.

16. Verwendung nach Anspruch 10, dadurch gekennzeichnet, daß man die Adsorbentien zur Herstellung von Gaselektroden verwendet, die eine Isolierung von Wasserstoff gestatten, der in einem Gemisch mit einem elektrolytischen Medium enthalten ist.

17. Verwendung nach Anspruch 11, dadurch gekennzeichnet, daß man das Adsorbens in der Form eines Überzuges verwendet, der wenigstens einen Teil der Elektrodenoberfläche bildet.

## Claims

1. Gasadsorbents comprising at least one paracyclophane (or PCP) trimer formed by three units, which units may be different form each other, having the formula RX, wherein R is a cyclic aromatic compound having 6 chain links substituted in each para position by X, said cyclic compound being selected from the group of radical phenylene-$C_6H_4$- or an aromatic nitrogen containing pyridinyl or pyrimidinyl radical, provided that the nitrogen atom or atoms do not occupy the para positions substituted by X, and X is a linear chain link having two elements, selected more especially from the radicals

$-CH_2-CH_2-$ 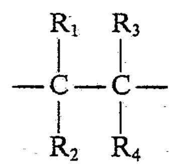 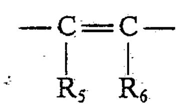 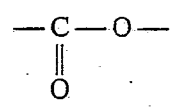 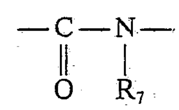

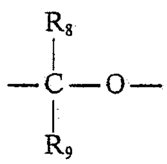 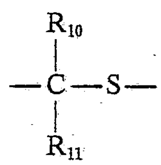 $-S-S-$ 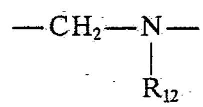 or 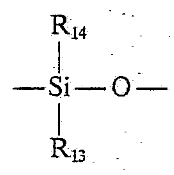

$R_1$ to $R_4$ are same or different groups $C_nH_{2n+1}$ with $1 \leq n \leq 4$ or a halogen atom selected from the group of F, Cl and Br and $R_5$ to $R_{14}$ may be a hydrogenatom additionally to the meaning stated for $R_1$ to $R_4$ characterized in that the PCP molecule is bound to a polymer chain.

2. Adsorbents according to claim 1, characterized in that they comprise at least one polymer chain of the type poly(p-vinyl)PCP.

3. Adsorbents according to claim 1, characterized in that they comprise a macromolecular structure to which PCP units are fixed.

4. Use of derivates of paracyclophane or PCP, consisting or cyclic oligomers formed by basic units which are connected in such a manner that they define a rigid structure of an intramolecular cavity substantially cylindric, the dimensions of the cross-section of said cavity corresponding substantially to those of the types of molecules of gas which are desired to be inserted, the rigidity of the structure being such that the variations of the cross-section of the cavity are at most 10% more or less, until a temperature in the order of 250° C as gasadsorbents.

5. Use according to claim 4, characterized in that the paracyclophane derivates comprise at least one PCP-trimer formed by three units which units may differ form each other, having the formula RX, wherein R is a cyclic aromatic compound having 6 chain links substituted in each para position by X, said cyclic compound being selected from the radical phenylene-$C_6H_4$- or an aromatic nitrogen containing pyridinyl or pyrimidyl radical, provided that the nitrogen atom or atoms do not occupy the para positions substituted by X, and X is a linear chain link having two elements selected more especially from the radicals

$-CH_2-CH_2-$ 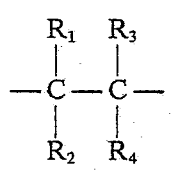 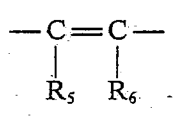 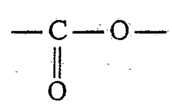 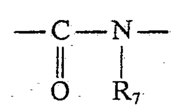

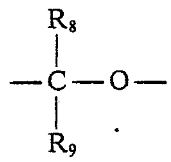 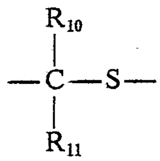 $-S-S-$ 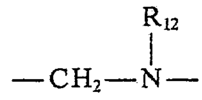 or 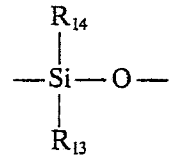

$R_1$ to $R_4$ being same or different from each other and being a group $C_nH_{2n+1}$ with $1 \leq n \leq 4$ or a halogen atom selected from the group F, Cl or Br, $R_5$ to $R_{14}$ representing a hydrogen atom additionally to the meanings shown for $R_1$ to $R_4$.

6. Use according to claim 5, characterized in that the paracyclophane derivates comprise at least one PCP-trimer wherein X is a chain link $-CH_2-CH_2-$.

7. Use according to claim 5, characterized in that the paracyclophane derivatives comprise at least one PCP-trimer wherein X is a functional ester group.

8. Use according to claim 4, characterized in that the paracyclophane derivatives include at least one cyclic tetramer formed by basic units $R-Y$, wherein R has the meaning stated above and Y is a chain link having one element selected form the group

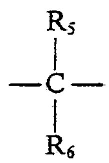

wherein $R_5$ and $R_6$ have the meaning stated above,

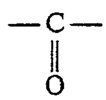 $-O-$ or $-S-$

9. Use according to claim 5, characterized in that the paracyclophane derivatives comprise at least one PCP-trimer formed by three units, one of the aromatic cycles of which being substituted by at least one element M selected from the group of the halogenes, especially F, Cl and Br, an acidic group such as $-COOH$ or $SO_3H$, an ammonium cation of the type $-N(R_{15})_3^+$, $R_{15}$ representing a radical $C_nH_{2n+1}$ with $0 \leq n' < 2$ or an ethylenically unsaturated group, advantageously the vinyl group.

10. Use of adsorbents according to claim 5 for the retention of hydrogen.

11. Use of adsorbents according to claim 8 for the retention of molecules more voluminous than hydrogen such as $O_2$ or $N_2$.

12. Use of adsorbents according to claim 10, characterized in that there is operated at temperatures near the ambient temperature and within a range of pressures of 5 to 100 bar, especially from 5 to 50 bar.

13. Use of adsorbents according to claim 3, characterized in that more especially for the purification of hydrogen and the concentration thereof, there is contacted a phase containing an efficient amount of at least one of the said adsorbents, with a gaseous or liquid phase containing hydrogen in a mixture with for example $O_2$, $N_2$ or $H_2O$.

14. Use according to claim 10, characterized in that the said adsorbent is used in the solid state, especially in the form of balls, granules or membranes, dispersed in an amorphous phase or a macromolecular material.

15. Use according to claim 10, characterized in that the adsorbent is used in the solid state, especially in the form of balls, granules or membranes, in dispersion or solution in a liquid, especially in a viscous liquid, more especially in a usual membrane material.

16. Use according to claim 10, characterized in that the adsorbents are used for the preparation of gas electrodes permitting the isolation of hydrogen contained in a mixture with an electrolytic medium.

17. Use according to claim 11, characterized in that the adsorbent is used in the form of a coating froming at least a part of the surface of the electrodes.